(19) Europäisches Patentamt
European Patent Office
Office européen des brevets

(11) **EP 4 589 538 A1**

(12) **EUROPEAN PATENT APPLICATION**

(43) Date of publication:
**23.07.2025  Bulletin 2025/30**

(21) Application number: **25151982.3**

(22) Date of filing: **15.01.2025**

(51) International Patent Classification (IPC):
**G06T 11/00** $^{(2006.01)}$   **A61B 6/00** $^{(2024.01)}$
**G06T 7/33** $^{(2017.01)}$

(52) Cooperative Patent Classification (CPC):
**G06T 11/005; A61B 6/027; A61B 6/032;
A61B 6/5258; A61B 6/5264; G06T 7/344;**
G06T 2207/10072; G06T 2211/40

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.01.2024  JP 2024006305**

(71) Applicant: **FUJIFILM Corporation
Tokyo Tokyo 106-8620 (JP)**

(72) Inventor: **YUSUKE, Tetsumura
Tokyo (JP)**

(74) Representative: **Dehns Germany Partnerschaft
mbB
Theresienstraße 6-8
80333 München (DE)**

(54) **X-RAY CT APPARATUS AND PROCESSOR FOR IMAGE PROCESSING**

(57)  Provided is an X-ray CT apparatus (1) capable of preventing excessive motion correction or deformation, particularly maintaining continuity of images in a body axis direction, and performing motion correction reconstruction under an optimal condition according to an image reconstruction condition, in motion correction reconstruction.

A processor (200) for image processing of an X-ray CT apparatus (1) according to the present invention includes a motion estimation model including regularization terms that are independently adjustable for continuity of a spatial domain and continuity of a time domain, as a motion estimation model for acquiring motion information. The motion estimation model is configured to adjust a weight of the regularization term and a control point position. A motion information acquisition unit automatically adjusts the motion estimation model or a calculation method of a control point parameter using the motion estimation model according to a FOV or an image reconstruction interval which is an image reconstruction condition. As a result, it is possible to satisfactorily maintain image continuity in one cross section and inter-slice image continuity in a body axis direction.

**FIG. 3**

Description

CROSS-REFERENCE TO RELATED APPLICATIONS

The present application claims priority under 35 U.S.C. § 119 to Japanese Patent Application No. 2024-006305, filed January 18, 2024. Each of the above application(s) is hereby expressly incorporated by reference, in its entirety, into the present application.

BACKGROUND OF THE INVENTION

1. Field of the Invention

The present invention relates to an X-ray CT apparatus and a processor that processes transmitted X-ray data obtained by the X-ray CT apparatus, and particularly relates to a motion correction reconstruction technique for a CT image.

2. Description of the Related Art

In an X-ray CT apparatus, a subject is disposed at an opening of a scanner equipped with an X-ray source and an X-ray detector, imaging is performed while the scanner is rotated, and an image of a cross section of the subject is reconstructed using X-ray projection data acquired at each angle of the scanner. In this case, the imaging is performed while moving a position of the subject in a body axis direction with respect to the scanner, thereby obtaining images of a plurality of cross sections along the body axis direction.

In imaging of a site of the subject having a motion, such as chest radiography using a CT apparatus, in order to reduce artifacts caused by the motion, motion correction reconstruction is widely adopted in which motion information of the subj ect during imaging is detected and image reconstruction is performed using the motion information.

For the detection of the motion information, a pair of partial angle reconstruction (PAR) images (hereinafter, referred to as PAR images) reconstructed using the transmitted X-ray data (hereinafter, referred to as projection data) of less than 180 degrees acquired at positions directly facing each other in time with a target image reconstruction position as a center is used (for example, US2018/0005414A). Specifically, two PAR images constituting the pair of PAR images are reconstructed from data obtained by projecting the subject from directions inverted by 180 degrees, and the two PAR images are the same image in a case where there is no motion of the subject while the scanner moves by 180 degrees, but there is a change due to a cardiac beat and a respiratory motion during the movement of the scanner. In the motion correction reconstruction, a change amount of each pixel of the PAR image is calculated as the motion information, and the image is reconstructed by performing correction based on the motion information on each projection data.

In cardiography targeting the heart, in order to minimize an influence of the cardiac beat, electrocardiography is performed in which the target image reconstruction position matches a specific phase of a cardiac phase. Therefore, the motion information obtained from the PAR image is also information corresponding to the specific phase. Meanwhile, in chest radiography (asynchronous imaging) in which electrocardiographic synchronization is not performed, a reconstruction center phase is different in images of the respective cross sections. Therefore, there is a problem in that continuity of the images is significantly reduced particularly in the body axis direction.

SUMMARY OF THE INVENTION

In the technique disclosed in US2018/0005414A, a free-form deformation model based on a 4D B-spline function in which time information upon acquisition of the PAR image is also taken into account is used as a motion estimation model in a case of acquiring the motion information using the PAR image, and a plurality of PAR image pairs are used to prevent deterioration of continuity of the images due to misalignment of the reconstruction center phases. However, in this technique, a plurality of PAR image pairs are used for reconstructing one cross section, and thus there is a problem of high computational costs. In addition, in this technique, the continuity of the images in the body axis direction is not considered.

In addition, in the asynchronous imaging, a table speed is usually faster to cause a sparse scan than in the electrocardiography, and an amount of data that can be used for PAR image creation is reduced. Therefore, there is a problem in that a motion correction accuracy is reduced.

Further, in a case where data continuously captured in the body axis direction is subjected to image reconstruction, image reconstruction conditions such as a slice thickness and a FOV may be changed depending on an imaging position. However, in the motion correction reconstruction technique in the related art, the image reconstruction conditions are highly dependent on the motion correction accuracy.

An object of the present invention is to maintain continuity of images particularly in a body axis direction in imaging using an X-ray CT apparatus and to enable optimal motion correction reconstruction regardless of image reconstruction conditions.

The present invention introduces a motion estimation model that can independently control each of continuity in a spatial domain and continuity in a time domain, as a motion estimation model for acquiring motion information. By independently controlling the continuity of the spatial domain and the continuity of the time domain, it is possible to satisfactorily maintain image continuity in one cross section and inter-slice image continuity in the body axis direction.

In addition, the present invention configures a motion estimation model or a calculation method using the motion estimation model to be dynamically adjustable with respect to a change in image reconstruction conditions.

That is, a first aspect of the present invention is the following X-ray CT apparatus.

The X-ray CT apparatus comprises: an imaging unit that has a scanner equipped with an X-ray source and an X-ray detector and rotating around a subject and a moving mechanism moving a position of the scanner in a body axis direction of the subject relative to the subject, and that acquires transmitted X-ray data (hereinafter, referred to as projection data) having different angles with respect to the subject and different positions in the body axis direction; and a computing unit that generates a tomographic image of the subject using the projection data acquired by the imaging unit.

The computing unit includes a partial reconstruction image generation unit that generates a pair of partial reconstruction images at positions directly facing each other using the projection data, a motion information acquisition unit that acquires motion information of the subject during scanning by applying a motion estimation model to the pair of partial reconstruction images, and a motion correction reconstruction unit that reconstructs the tomographic image using the motion information and the projection data acquired in an angle range of 180 degrees or more. The motion estimation model includes a first regularization term that maintains spatial continuity of an image and a second regularization term that maintains temporal continuity of the image, which are independently adjustable. The motion information acquisition unit automatically changes at least one of the motion estimation model or a calculation method of motion estimation using the motion estimation model according to an image reconstruction condition.

In addition, a second aspect of the present invention is a processor for image processing, the processor being configured to: generate a pair of partial reconstruction images at positions directly facing each other using projection data; acquire motion information of a subject during scanning by applying a motion estimation model including a first regularization term that maintains spatial continuity of an image and a second regularization term that maintains temporal continuity of the image, which are independently adjustable, to the pair of partial reconstruction images; and reconstruct a tomographic image using the motion information and the projection data acquired in an angle range of 180 degrees or more.

According to the present invention, since the motion estimation model including the first regularization term that maintains the spatial continuity of the image and the second regularization term that maintains the temporal continuity of the image, which are independently adjustable, is provided, it is possible to adjust the spatial and temporal continuity of the image in response to the set image reconstruction condition or a change in the image reconstruction condition, and thus it is possible to improve an accuracy of the motion estimation. In addition, according to the present invention, it is possible to dynamically change motion estimation processing using the motion estimation model, and even in a case where the image reconstruction condition is changed during the image reconstruction, it is possible to maintain the continuity of the image particularly in the body axis direction, thereby preventing deformation due to excessive motion correction and providing an image that contributes to diagnosis.

## BRIEF DESCRIPTION OF THE DRAWINGS

Fig. 1 is a diagram showing an overall outline of an X-ray CT apparatus to which the present invention is applied.
Fig. 2 is a functional block diagram of an X-ray CT apparatus according to an embodiment of the present invention.
Fig. 3 is a functional block diagram of an embodiment of a processor provided in the X-ray CT apparatus.
Fig. 4 is a diagram showing an outline of motion correction reconstruction processing common to the respective embodiments of the present invention.
Fig. 5 is a diagram illustrating the motion correction reconstruction.
Fig. 6 is a diagram illustrating generation of a partial reconstruction image.
Fig. 7 is a diagram illustrating a concept of a motion estimation model of Embodiment 1.
Fig. 8 is a diagram illustrating processing of Embodiment 2 and showing a relationship between a FOV and a control point interval.
Fig. 9 is a diagram illustrating the processing of Embodiment 2 and showing a relationship between the FOV and a control point position.
Fig. 10 is a diagram showing a flow of processing of Embodiment 3.
Fig. 11 is a diagram illustrating the processing of Embodiment 3.

**DESCRIPTION OF THE PREFERRED EMBODIMENTS**

Hereinafter, embodiments of an X-ray CT apparatus and a processor that is mainly responsible for image processing according to the present invention will be described. In the present embodiment, the processor means hardware including a general-purpose computer comprising a general-purpose central processing unit (CPU) or a general-purpose graphic processing unit (GPU) and a memory, and a programmable integrated circuit (IC) such as an application specific integrated circuit (ASIC), a field programable gate array (FPGA), or a complex programmable logic device (CPLD), and any one or a combination thereof is collectively referred to as a processor.

First, an overall configuration of an X-ray CT apparatus to which the present invention is applied will be described.

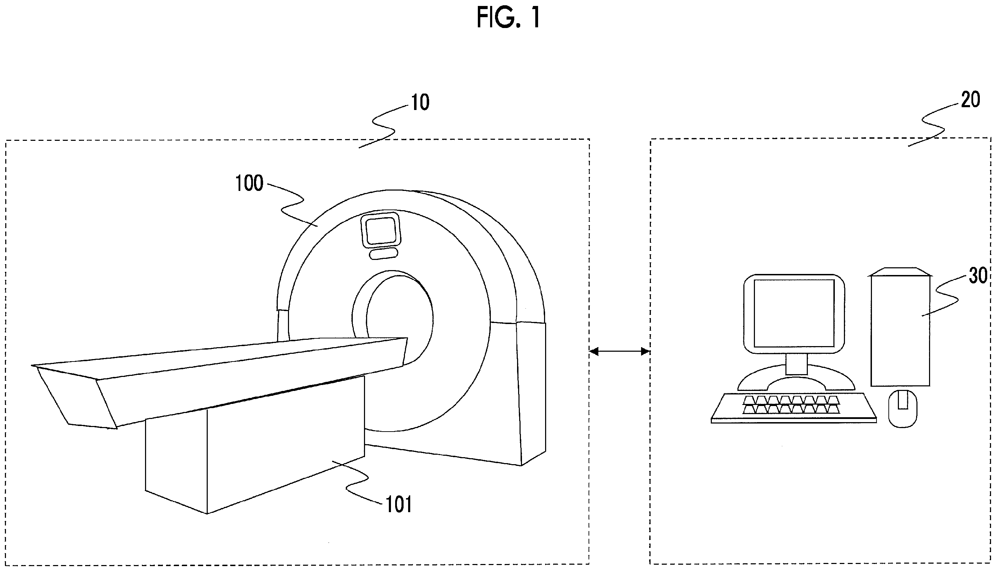

As shown in Fig. 1, an X-ray CT apparatus 1 comprises an imaging unit 10 comprising a gantry 100 and a couch device 101 for capturing a tomographic image and a fluoroscopic image of a subject 3, and an operation unit 20 that operates and controls the imaging unit 10.

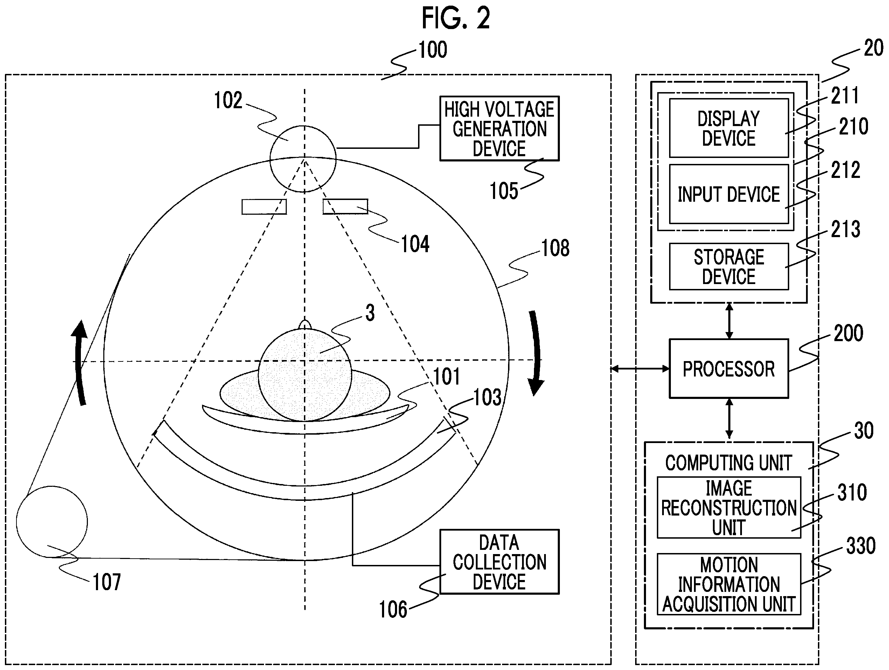

As shown in Fig. 2, the gantry 100 comprises an X-ray generation device 102 that generates X-rays with which the subject 3 is irradiated, a collimator device 104 that narrows a flux of the X-rays generated from the X-ray generation device 102, an X-ray detection device 103 that detects the X-rays transmitted through the subject, a scanner 108 on which the X-ray generation device 102, the collimator device 104, and the X-ray detection device 103 are mounted, a high voltage generation device 105 that applies a high voltage to the X-ray generation device 102, a data collection device 106 that collects transmitted X-ray data (referred to as projection data) obtained from the X-ray detection device 103, and a drive device 107 that rotates the scanner around the subject 3. The X-ray generation device 102 comprises an X-ray tube (not shown), and the subject 3 is irradiated with a predetermined amount of the X-rays by a predetermined tube current flowing through the X-ray tube.

The operation unit 20 comprises a processor 200 that functions as a central control device controlling each device built in the gantry, and an input/output device 210 that functions as a user interface for performing communication between a user and the processor 200. The processor 200 is equipped with a computing unit 30 that performs various types of computing, such as image reconstruction, on the projection data collected by the data collection device 106. Note that a processor that is separate from the processor 200 and independent of the X-ray CT apparatus and that processes data from the X-ray CT apparatus can also function as the computing unit 30, and such an independent processor is also included in the present invention. The function of the processor 200 is realized by the processor 200 reading and executing a program that describes a computing algorithm or a processing procedure for control, but a part of the computing or the processing performed by the computing unit 30 can also be performed by using a programmable logic device (PLD), such as an ASIC or an FPGA.

The input/output device 210 includes an input device 212 that allows an operator to input imaging conditions and the like, a display device 211 that displays data, such as a captured image, or a GUI, and a storage device 213 that stores data necessary for imaging, such as a program or a device parameter.

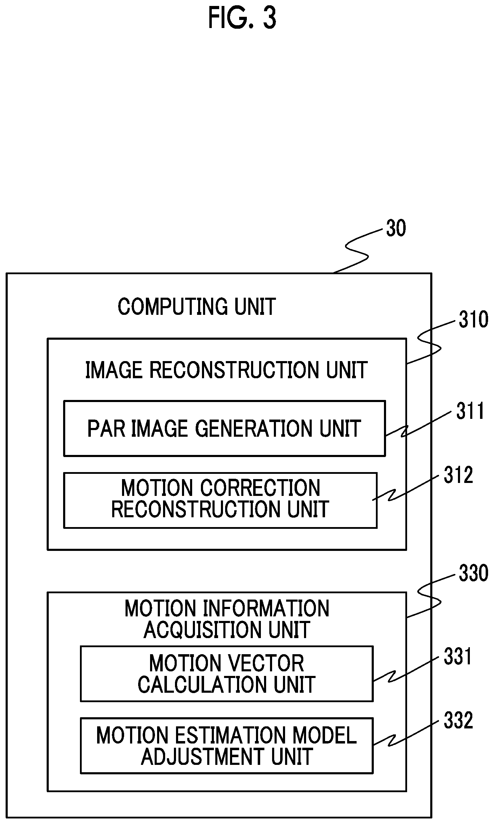

The computing unit 30 comprises an image reconstruction unit 310 that performs back projection processing on the projection data obtained by the data collection device 106 to create the tomographic image, and a motion information acquisition unit 330 that detects a motion of the subject being scanned. Further, as shown in Fig. 3, the image reconstruction unit 310 has a function of creating a partial reconstruction image (PAR image) using the transmitted X-ray data in an angle range of less than 180 degrees (PAR image generation unit 311), and a function of performing motion correction reconstruction using motion information acquired by the motion information acquisition unit 303 (motion correction reconstruction unit 312), in addition to a function of creating the tomographic image using the transmitted X-ray data in an angle range of 180 degrees or more. The motion information acquisition unit 330 has a function of calculating a motion vector (also referred to as a displacement vector) representing the motion of the subject using a pair of the PAR images, that is, the PAR images having different projection data acquisition angle ranges, created by the PAR image generation unit 311 and a predetermined motion estimation model (motion vector calculation unit 331), and a function of optimizing the motion estimation model in accordance with reconstruction conditions (motion estimation model adjustment unit 332).

The processor 200 operates as a processor that implements the function as the computing unit 30 described above, and controls the imaging unit 10 (the X-ray generation device 102, the X-ray detection device 103, the high voltage generation device 105, the collimator device 104, the couch device 101, the drive device 107, and the data collection device 106), the input/output device 210, and the computing unit 30 in response to an operation instruction from an operator through the input device 212. Each of these units operates under the control of the processor 200 to reconstruct a CT image, and to display and store the reconstructed CT image.

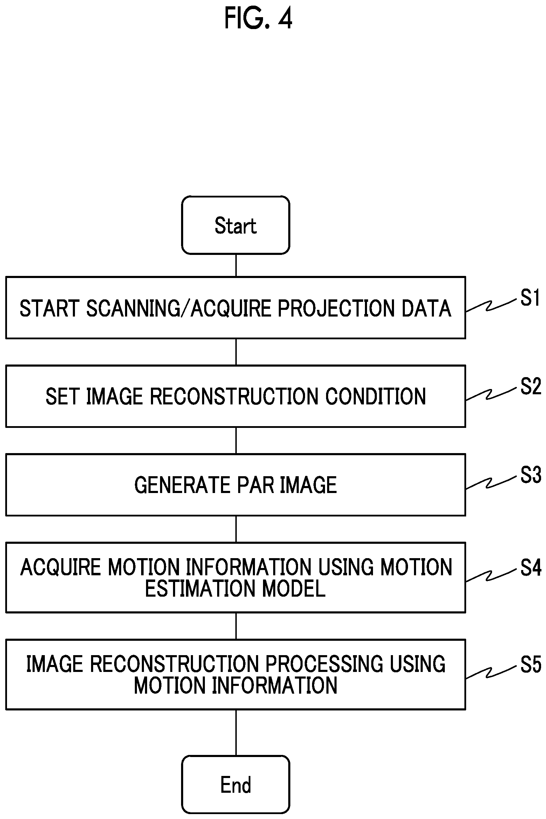

An outline of processing in a case where imaging is performed on a site having a motion and a motion correction is performed in the X-ray CT apparatus having the above-described configuration will be described with reference to Fig. 4.

S1

The subject 3 is placed on the couch device 101, positioning imaging is performed to set an imaging range (region of the subject in the body axis direction), and then the imaging unit 10 starts scanning the imaging range with the rotation of the scanner 108. The data collection device 106 collects the projection data acquired by the X-ray detection device 103 at each rotation angle and transmits the projection data to the processor 200.

S2

The user sets an image reconstruction condition for the projection data collected in the imaging step S1. The image reconstruction condition includes, for example, conditions such as a type or a parameter of a reconstruction filter used for the reconstruction, an image reconstruction interval, a field of view (FOV), and application or non-application of a motion correction function.

S3

The computing unit 30 generates a tomographic image according to the image reconstruction condition set by the user using the projection data collected by the data collection device 106. In a case where the motion correction function is not applied, the image reconstruction unit 310 performs image reconstruction by a method such as filtered back projection according to the set image reconstruction condition. On the other hand, in a case where the application of the motion correction is selected as the image reconstruction condition, the PAR image generation unit 311 generates PAR images at positions symmetrical to each other with respect to a target reconstruction center.

S4

The motion information acquisition unit 330 performs processing of estimating the motion of the subject by non-rigid registration using the PAR image, that is, acquisition of the motion information. The motion estimation (non-rigid registration) by the motion information acquisition unit 330 is a computation using a motion estimation model (B-spline function) performed mainly by the motion vector calculation unit 331, and a motion vector field (MVF) in which a vector of a motion of each pixel (control point) is mapped is obtained as the motion information.

The motion information acquisition unit 330 dynamically adjusts the motion estimation model and a parameter or a calculation method used in the computation, in accordance with the image reconstruction condition at a time point of applying the motion estimation model, to maintain spatial and temporal image continuity between the images (processing of the motion estimation model adjustment unit 332). The adjustment of the motion estimation model will be described in detail in embodiments described below.

S5

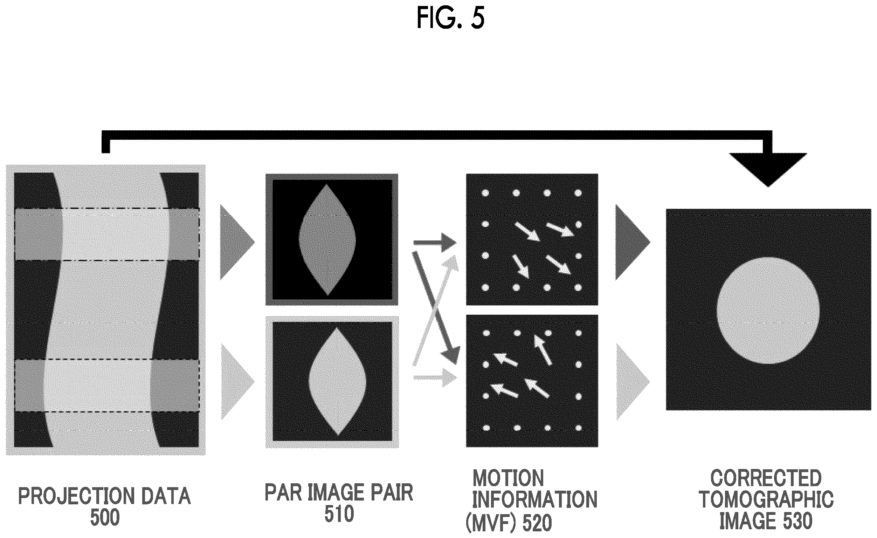

The motion correction reconstruction unit 312 performs image reconstruction of the projection data obtained in a range of 180 degrees or more using the motion information (MVF) acquired by the motion information acquisition unit 330. A method for motion correction image reconstruction is the same as a known method, and as shown in Fig. 5, the motion correction reconstruction unit 312 estimates a magnitude and a direction of the motion of the subject at the time of acquiring each projection data 500 used for image reconstruction from the motion information (MVF) 520 obtained from the PAR image pair 510, and performs filtered back projection while correcting an image at a target reconstruction position using an estimation result, thereby reconstructing a tomographic image 530.

The reconstruction of the tomographic image (steps S3 to S5) is executed at the image reconstruction interval set as the image reconstruction condition, and a plurality of 2D tomographic image data or 3D tomographic image data are obtained along the body axis direction. The image reconstruction unit 310 displays the tomographic image data on the input/output device 210 (display device 211) as an image in a predetermined display format.

In consideration of the configuration of the X-ray CT apparatus and the outline of processing described above, hereinafter, embodiments of the motion correction reconstruction performed by the processor 200, in particular, the adjustment of the motion correction model will be described. In the following embodiments, as an example, a case will be described in which a plurality of cross-sectional images along the body axis direction are captured asynchronously for a site having a motion, such as a chest.

Embodiment 1

The present embodiment is characterized in that a free-form deformation model (FFD) based on a 3D B-spline function is used as the motion estimation model, and a change is made to the model in consideration of image continuity. For the continuity of the image, information on the spatial domain and information on the time domain are introduced independently of each other to achieve the continuity of the image not only within the tomographic image but also along the body axis direction.

Hereinafter, processing of the present embodiment, particularly processing of motion estimation (S3 and S4 in Fig. 4) will be described. In the following description, Fig. 4 will be referred to as necessary.

S1 and S2

The imaging unit 10 scans the imaging range to collect transmitted X-ray data at each angle of the scanner. The image reconstruction condition is set by the user through the input/output device 210.

Processing of PAR Image Generation Unit: S3

In a case where the application of the motion correction function is selected as the image reconstruction condition, the PAR image generation unit 311 generates two partial reconstruction images (PAR images) from the projection data collected by the data collection device 106 by using a filtered back projection method. The two PAR images are partial images generated using projection data in an angle range of less than 180 degrees acquired at positions directly facing each other with a target reconstruction image position as a center. The target reconstruction image position is a position where the scanner is at a predetermined rotation angle (for example, a position at 0 degrees where the X-ray source is directly above the subject) and can be set by the user in a case of setting the image reconstruction condition, or a default value can be changed.

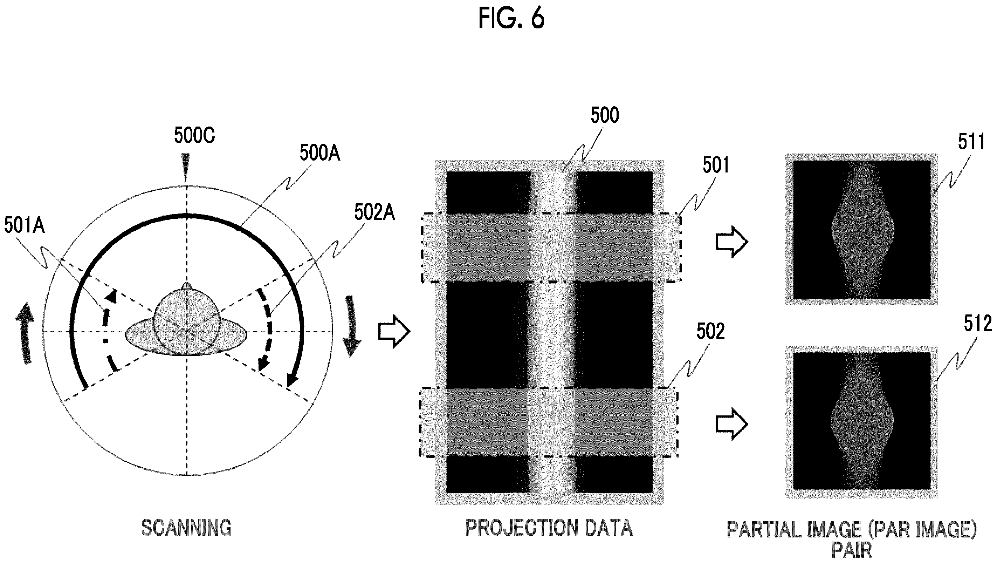

A relationship between the rotation angle of the scanner, the projection data, and the PAR image pair is shown in Fig. 6. The projection data 500 at a center of the drawing is projection data collected in an angle range 500A of 180 degrees or more of the scanner, and projection data 501 and 502 collected in the same angle ranges 501A and 502A directly facing each other with respect to an image reconstruction center position 500C among the projection data 500 are back-projected, respectively, to obtain partial images 511 and 512 as shown on the right side of Fig. 5. The partial images use the projection data in an angle range of less than 180 degrees is used and thus are not complete cross-sectional images. However, each pixel has positional information from which the motion information can be estimated.

The PAR image need not be a two-dimensional image, but may be a three-dimensional image.

In a case where a plurality of cross sections are imaged along the body axis direction, the PAR image generation unit 311 generates a PAR image for each cross section.

Processing of Motion Vector Calculation Unit: S4

The motion information acquisition unit 330 estimates the motion of the subject while the scanner is moved from a position of 501A to a position of 502A by using the PAR image pair generated by the PAR image generation unit 311. Before the motion estimation, filtering for noise reduction may be performed on the two PAR images as necessary. By performing the noise reduction processing, it is possible to improve an accuracy of subsequent motion estimation processing. A smoothing filter such as a bilateral filter can be used as a filter, and a degree of noise reduction can be adjusted by adjusting a parameter thereof. In a case where the projection data (511 and 512 in Fig. 6) of the two PAR images are data at symmetrical positions with respect to a rotation angle of 0 degrees of the scanner, generally, conditions of the tube current are the same, and degrees of noise are also the same. However, in a case where the conditions of the tube current are different, the parameters of the filter to be applied may be different according to a magnitude of the tube current in a case of acquiring the projection data.

In the estimation of the motion, non-rigid registration is performed between a pixel of interest of the partial image 511 and a pixel of the partial image 512 corresponding to the pixel of interest, and a motion vector field (MVF) between the images is calculated. The non-rigid registration is processing of calculating the MVF using the motion estimation model (function), and in the present embodiment, a free-form deformation (FFD) model based on a 3D B-spline function is used as the motion estimation model. The FFD model based on the 3D B-spline function is represented by, for example, the following Equation (1).

$$T_{t,j}(x; \Theta) = x + \sum_{j} B\left(\frac{x}{d} - j\right) \Theta_{t,j}$$

$$(1)$$

In the equation, $T_{t,j}$ represents FFD, x represents any voxel, t represents a time point of interest, j represents a control point of interest, B represents a cubic B-spline third-order tensor product, d represents an interval between control points in a spatial domain, and $\Theta_{t,j}$ represents a displacement vector (3D) of the control point at a time point t and a position j, and $\Theta$ represents a set (MVF: motion vector field) of displacement vectors (3D) of the control points representing a relationship between a reference time point and a motion at each time point.

The motion estimation processing results in calculating $\Theta$ (control point parameter), which is a parameter for changing the control point of the motion estimation model. The control point parameter $\Theta$ can be obtained by minimizing a dissimilarity (next Equation (2)) based on a sum of squared differences (SSD) between the PAR image at the reference time point and the PAR image at an angle facing an angle at the reference time point.

$$D(\Theta) = \sum_x \left| P_{target}(x) - P_{source}\left(T_j(x; \Theta)\right) \right|^2 \tag{2}$$

In the equation, D(O) is a dissimilarity based on the SSD, $P_{target}$ is a PAR image at the reference time point, and $P_{source}$ is a PAR image at an angle (time point) separated from the reference time point by 180 degrees.

Since a convergence calculation for the minimization is an ill-posed problem having a large number of transformation parameters, a regularization term is introduced in order to solve the problem efficiently and robustly. The regularization term is introduced as a term that penalizes a difference between control point parameters, but in the present embodiment, as shown in Equations (3) and (4), a regularization term of a spatial domain (first regularization term) and a regularization term of a time domain (second regularization term) are introduced as independent regularization terms. Equation (3) is a regularization term $R_1(\Theta)$ that penalizes a difference between control point parameters that are spatially adjacent to each other, and is a regularization term for suppressing excessive deformation in the image of interest. Equation (4) is a regularization term $R_2(\Theta)$ that penalizes a difference between control point parameters that are temporally adjacent to each other, and is a term for suppressing excessive deformation from an image adjacent to the image of interest. The images that are temporally adjacent are the image of interest and images before and after the image of interest (before and after in time, that is, before and after in the body axis direction).

$$R_1(\Theta) = \sum_t \sum_{j' \in K_j} \left\| \theta_{t,j} - \theta_{t,j'} \right\|_2^2 \tag{3}$$

$$R_2(\Theta) = \sum_{t' \in K_t} \sum_j \left\| \theta_{t,j} - \theta_{t',j} \right\|_2^2 \tag{4}$$

In a case where these regularization terms are used, a cost function of the control point parameters is finally represented by the following Equation (5).

$$C(\Theta) = D(\Theta) + \lambda_1 R_1(\Theta) + \lambda_2 R_2(\Theta)$$

$$= \sum_x \left| P_{target}(x) - P_{source}\left(T_j(x;\Theta)\right) \right|^2$$

$$+ \lambda_1 \sum_j \sum_{j' \in K_j} \left\| \theta_{t,j} - \theta_{t,j'} \right\|_2^2$$

$$+ \lambda_2 \sum_{t' \in K_t} \sum_j \left\| \theta_{t,j} - \theta_{t',j} \right\|_2^2 \tag{5}$$

In the equation, $\lambda_1$ and $\lambda_2$ are weights of two regularization terms $R_1(\Theta)$ and $R_2(\Theta)$, respectively.

By introducing such two regularization terms, the motion vector calculation unit 331 can stably find an optimal solution for minimizing Equation (2), and the motion vector $\Theta$ is obtained.

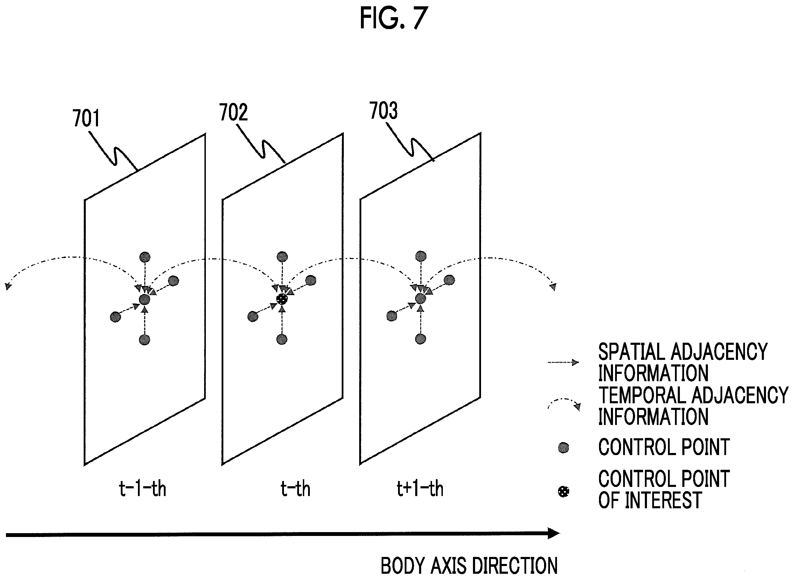

Fig. 7 shows a concept of the motion estimation model in which the regularization term is introduced. As shown in Fig. 7, in a case where a plurality of tomographic images, that is, a t-1-th image 701, a t-th image 702, and a t+1-th image 703 are reconstructed at predetermined image reconstruction intervals in the body axis direction, and a central point of the t-th image 702 is set as a control point of interest, in the motion estimation model, a parameter of the control point of interest is determined by taking information (spatial adjacency information) on control points adjacent to the control point of interest in the image 702 and information (temporal adjacency information) on control points of the images 701 and 703 corresponding to the control point of interest of the image 702 into consideration.

Therefore, the continuity of the images depends on an interval between the control points in the image and an interval between the control points between the adjacent images. In addition, the continuity of the images also depends on magnitudes of the weights of the two regularization terms described above. The motion estimation model of the present embodiment is characterized in that the control point and the weight of the regularization term are provided to be adjustable according to the image reconstruction condition such as the FOV or the image reconstruction interval, and thus the continuity of the images can be optimized.

A specific example of the adjustment will be described in the following embodiment. For example, in the adjustment of the control point, the number of pixels or the position (coordinates) on the image that determines the interval between the control points is changed according to the image reconstruction condition (for example, FOV). Regarding the weight of each regularization term, for example, a predetermined value is set in advance as a default for a standard image reconstruction condition (FOV or image reconstruction interval), and the weight $\lambda_1$ of the regularization term $R_1(\Theta)$ of the spatial domain or the weight $\lambda_2$ of the regularization term $R_2(\Theta)$ of the time domain is adjusted in a case where the FOV or the image reconstruction interval set by the user is different from the standard FOV or image reconstruction interval. The adjustment of the weight according to the image reconstruction condition can be dynamically performed not only in a case where the user sets the image reconstruction condition but also in response to changes in the image reconstruction condition during imaging.

Motion Correction Reconstruction Processing: S5

The motion correction reconstruction unit 312 performs image reconstruction by using a motion estimation result using the motion model described above. As shown in Fig. 5, in the motion correction reconstruction, the magnitude and the direction of the motion of the subject at the time of acquiring each projection data used for the image reconstruction are estimated from the MVF 520 calculated by using the PAR image pair 510 by the motion information acquisition unit 330, and the filtered back projection is performed while correcting the image at the target reconstruction position using the estimation result, thereby reconstructing the tomographic image 530.

As described above, with the X-ray CT apparatus of the present embodiment, by introducing the regularization terms of the spatial domain and the time domain that are independently adjustable as the motion estimation model in a case where the computing unit that performs the motion correction reconstruction performs the motion estimation process, it is possible to reduce an occurrence frequency of unnatural deformation and to provide a reconstructed image in which the continuity of the images is maintained particularly in the body axis direction.

In addition, according to the present embodiment, since the estimation of the motion uses only a pair of PAR images for one cross section, an amount of calculation is reduced, and a processing time and a memory usage can be reduced.

Next, a specific embodiment of the adjustment of the motion estimation model according to the image reconstruction condition will be described based on Embodiment 1 described above. The adjustment of the motion estimation model includes adjustment of the equation for calculating the control parameter (displacement vector) between the images described above, specifically, adjustment of weights of two regularization terms (a regularization term of a spatial domain and a regularization term of a time domain) in the calculation equation, adjustment of control points, adjustment of a calculation method, and the like, and the adjustment is performed in an aspect according to the image reconstruction condition.

Embodiment 2

In the present embodiment, the adjustment of the motion estimation model according to the FOV among the image reconstruction conditions will be described. A flow of processing is the same as the flow shown in Fig. 4, but in a case where the image reconstruction condition is set in S2 of the flow of Fig. 4, the motion estimation model adjustment unit 332 automatically adjusts the number of pixels between the control points (the number of pixels between the control point of interest and the next control point) based on the set FOV

Specifically, in a case where a FOV ($FOV_{adj}$) set by the user is different from a reference FOV ($FOV_{base}$), the motion estimation model adjustment unit 332 adjusts the number of pixels between control points ($INT_{adj}$) such that the number of pixels between control points ($INT_{base}$) of the $FOV_{base}$ is used as a reference and the control point interval (mm) is equal to the control point interval (mm) of the $FOV_{base}$. That is, the number of pixels between control points calculated by the following equation is set as the number of pixels between control points ($INT_{adj}$) of the set $FOV_{adj}$.

$$INT_{adj} = INT_{base} \times \frac{FOV_{base}}{FOV_{adj}} \qquad (6)$$

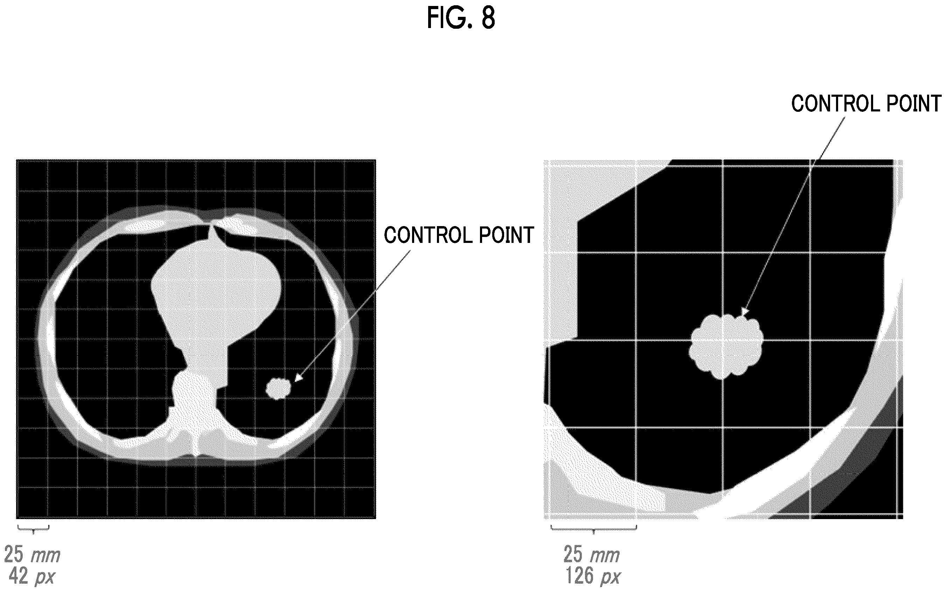

As an example, Fig. 8 shows a case where the reference $FOV_{base}$ is 300 mm, and the set $FOV_{adj}$ is 1/3 (100 mm) of 300 mm. Assuming that the control point interval is 25 mm ($INT_{base}$: 42 pixels) in a case of an image having a $FOV_{base}$ of 300 mm shown in a left diagram in Fig. 8, the number of pixels having the same control point interval is three times (126) in an image having a $FOV_{adj}$ = 100 mm in a right diagram. By the adjustment using Equation (6) described above, it is possible to prevent excessive deformation caused by the control point interval on a real image being too small. In addition, it is possible to perform motion estimation equivalent to the motion estimation in the reference $FOV_{base}$ without unnecessarily increasing the number of control points. In the example of Fig. 8, the reference $FOV_{base}$ is set to a size that covers the scanned subject range, but the reference $FOV_{base}$ may be determined to be a different value for each imaging site. For example, the reference values are set as FOV = 300 mm for the chest and FOV = 150 mm for the heart. In such a case, the $FOV_{adj}$ set later may be larger than the reference $FOV_{base}$, but the number of pixels between control points may be adjusted to be small such that the control point interval (mm) is kept the same according to Equation (6).

According to the present embodiment, even in a case where the user sets the FOV different from the reference FOV, the number of pixels between control points is adjusted according to the FOV, so that a degree of deformation is prevented from being changed depending on the FOV, and excessive deformation or insufficient deformation can be eliminated. That is, it is possible to improve an accuracy of the estimation of the motion and to improve an effectiveness of the motion correction reconstruction.

In the above description, a case where the image reconstruction condition set by the user in S 1 is different from the reference FOV set as a default condition of the image reconstruction condition has been described. However, the present disclosure can also be applied to a case where the user setting is changed during the image reconstruction, such as a case where a lesion of interest such as a nodule is found while observing images of both lungs with a large FOV (300 mm), and the FOV is enlarged to 150 mm to perform the enlarged image reconstruction. In such a case, by dynamically changing contents of the motion estimation processing according to the change in the image reconstruction condition, it is possible to maintain the continuity with the motion estimation processing so far and to prevent excessive deformation or the like caused by the change in the condition.

Modification Example 1 of Embodiment 2

In Embodiment 2, the number of pixels between control points in a case of motion estimation is adjusted according to the image reconstruction condition (FOV) set by the user. However, in the present modification example, an adjustment is made to fix the position of the control point on the image without depending on the FOV That is, even in a case where the FOV is changed, coordinates of the control point are controlled such that the position of the control point with respect to a center of the image is not changed. In this example, a case where a change is made to reduce the FOV during the image reconstruction with respect to the initially set FOV will be described as an example.

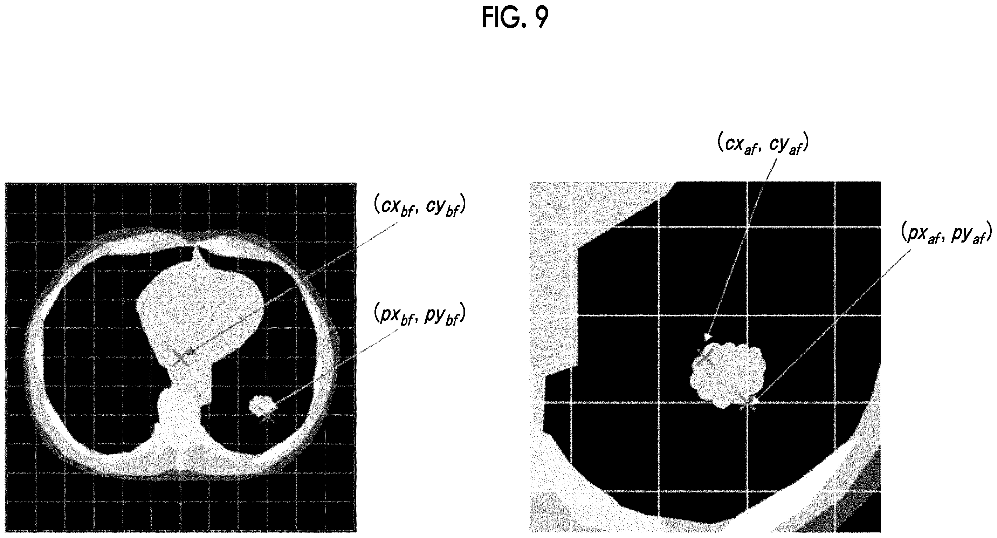

As shown in Fig. 9, in a case where coordinates of the control point of interest at a certain time point of the motion estimation processing are set to $(px_{bf}, py_{bf})$, reconstruction center coordinates are set to $(cx_{bf}, cy_{bf})$, and the FOV $(FOV_{bf})$ at the certain time point is changed to the $FOV_{af}$ as shown in a right diagram of Fig. 9, the motion estimation model adjustment unit 332 receives the change and then determines coordinates of the control point of interest according to the following equation for the image to be reconstructed next.

$$(px_{af}, py_{af}) = \left( \frac{FOV_{bf}}{FOV_{af}} (px_{bf} - cx_{bf}) + cx_{af}, \frac{FOV_{bf}}{FOV_{af}} (py_{bf} - cy_{bf}) + cy_{af} \right) \tag{7}$$

By this processing, the coordinates of each control point are always the same coordinates on the image, that is, fixed even in a case where the FOV of the reconstruction is changed. Therefore, it is possible to prevent fluctuation in the motion estimation accuracy caused by the change in the FOV and to realize the motion estimation with a certain accuracy.

Modification Example 2 of Embodiment 2

In Embodiment 2 and Modification Example 1, the number of pixels between control points or the coordinates of the control points upon motion estimation are adjusted according to the FOV However, instead of adjusting the control points, the weight $\lambda_1$ of the regularization term $R_1(\Theta)$ of the spatial domain in Equation (5) for calculating the displacement vector may be changed (adjustment of the calculation equation for motion estimation). For example, in a case where the FOV smaller than the reference is set with respect to the $\lambda_{1base}$ set as the default for the reference FOV, the weight $\lambda_1$ is set as $\lambda_1 > \lambda_{1base}$. By increasing the weight of the regularization term in the spatial domain, a penalty for excessive change (motion estimation) in the spatial domain caused by the reduction of the FOV can be increased, and the accuracy of the motion estimation can be improved. In contrast, in a case where the FOV larger than the reference FOV is set, the weight $\lambda_1$ is set as $\lambda_1 < \lambda_{1base}$. In this case as well, it is possible to prevent a fine motion caused by the increase of the FOV from being overlooked and to improve the accuracy of the motion estimation.

In addition, the method of Modification Example 2 can also be used in combination with the method of Embodiment 2, that is, adjusting the number of pixels between control points, or the method of Modification Example 1, that is, fixing the control point coordinates.

According to the present modification example, as in Embodiment 2 or Modification Example 1 thereof, it is possible to prevent excessive motion estimation or a decrease in the accuracy of the motion estimation due to the change of the FOV and to improve the accuracy of the motion estimation.

Embodiment 3

In the present embodiment, the same motion estimation model as in Embodiments 1 and 2 is used. Note that in Embodiment 2 and the modification examples thereof, the control point of the motion estimation model or the weight of the regularization term is adjusted in accordance with the change of the FOV However, in the present embodiment, a calculation method for the control parameter between images is dynamically changed so as not to be affected by the change in the image reconstruction interval.

In a case where the tomographic image is reconstructed at a predetermined image reconstruction interval in the body axis direction, processing of the motion estimation is to determine a control point parameter of an image adjacent to a reference image. The image reconstruction intervals of the images 701 to 703 shown in Fig. 7 are set as the image reconstruction conditions. However, in a case where the image reconstruction interval is changed, the control point interval in the body axis direction will differ.

In the present embodiment, the control parameter calculation method is adjusted such that the motion estimation is performed at a constant control point interval even in a case where the image reconstruction interval is changed. Therefore, in the present embodiment, it is assumed that there is a virtual tomographic image between adjacent images, and the motion estimation between the virtual tomographic images is calculated. That is, a provisional control point parameter is sequentially calculated for the virtual tomographic images, and in a case where a position of the virtual tomographic image reaches a position of an adj acent tomographic image, the provisional control point parameter calculated for the virtual tomographic image is set as the control point parameter of the adjacent tomographic image.

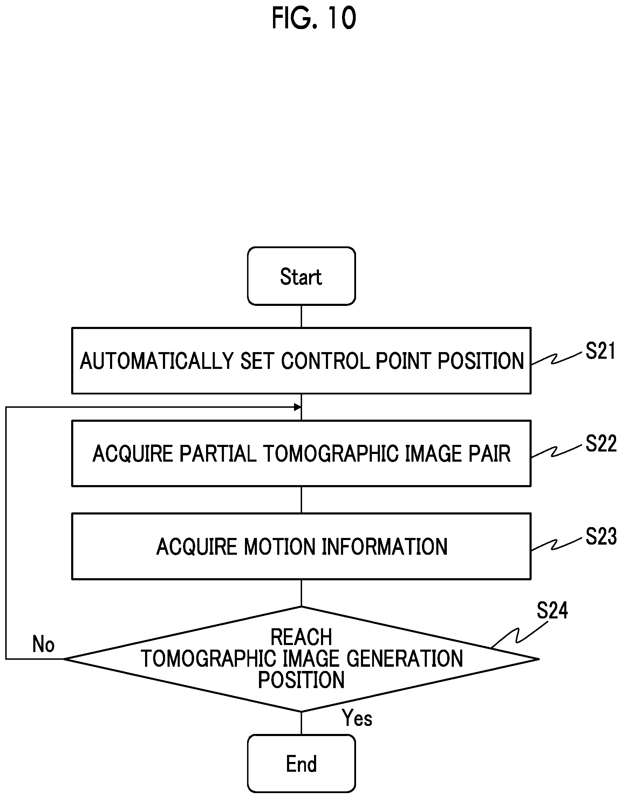

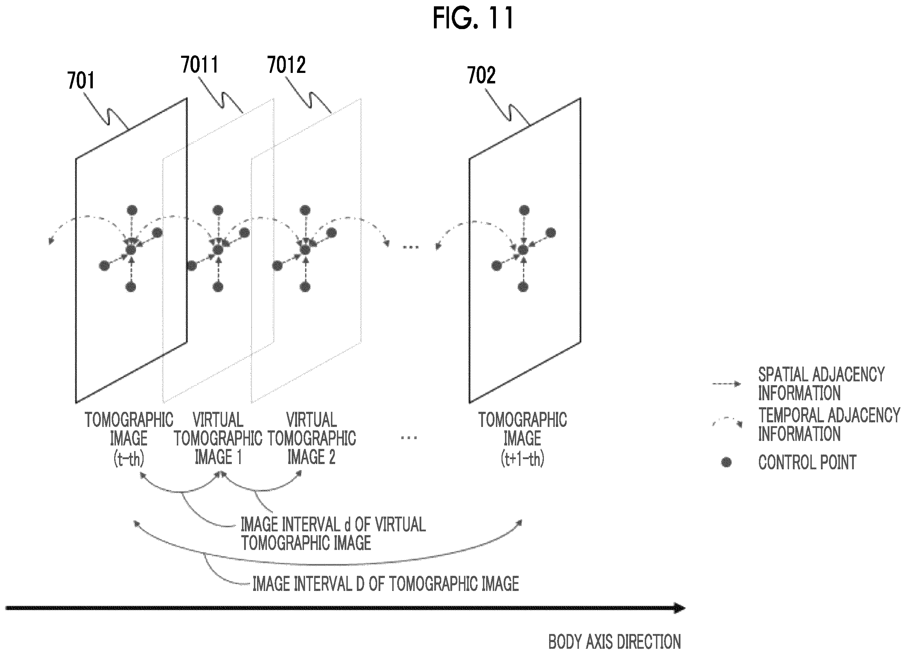

Hereinafter, the processing of the present embodiment will be specifically described with reference to Figs. 10 and 11.

Fig. 10 is a diagram showing processing of the present embodiment. As shown in Fig. 10, in the present embodiment, first, a control point position is automatically set (S21), and then the PAR image is generated for the virtual tomographic image (S22).

In the automatic setting of the control point position, for the control point in the tomographic image, the control point interval (interval in terms of the number of pixels) or the coordinates of the control point is set in accordance with the FOV set as the image reconstruction condition by the method of Embodiment 2 or Modification Example 1 thereof. That is, in a case where the FOV is smaller than the FOV set in advance, the number of pixels between the control points is changed such that a distance (mm) between the control points in a real space is constant (method of Embodiment 2). Alternatively, the control is performed such that, even for the image after the change of the FOV change, the position of the control point with respect to the image center is the same as that before the change of the FOV (method of Modification Example 1).

In the body axis direction, as shown in Fig. 11, a virtual tomographic image 7011 is assumed at a position at a predetermined interval d from a tomographic image 701 of interest. The interval d is a value smaller than a set image reconstruction interval D, and the interval d is a control point interval in the body axis direction in the present embodiment. For example, in a case where a standard image reconstruction interval D is set to 5 mm, the interval d is set to a value, such as a fraction thereof (for example, 1/10), that can respond to a case where a small image reconstruction interval such as 0.625 mm is set for more detailed diagnosis. This value d may be set to a predetermined value in advance, or may be set as a ratio to the set image reconstruction interval. In addition, a configuration set by the user may be adopted.

In a case where a position of the virtual tomographic image 7011 is determined, the PAR image generation unit 311 reconstructs the PAR image from a pair of projection data obtained in a predetermined angle range with respect to a reconstruction center position of the virtual tomographic image.

Then, the motion information acquisition unit 330 calculates the control point parameter using the motion estimation model (Equation (5)) for the virtual tomographic image using the PAR image to acquire the motion information (S23). The computing unit 30 sequentially calculates the control point parameters for the virtual tomographic images 7012 ... while changing the virtual tomographic image position in the body axis direction until an image reconstruction position of the adjacent tomographic images 702 is reached, for the PAR image generation (S22) and motion information acquisition (S23) described above. In a case where the position of the virtual tomographic image reaches the image reconstruction position (S24), the control point parameter calculated for the virtual tomographic image is set as the control point parameter of the adjacent image.

The motion correction reconstruction unit 312 reconstructs the adjacent tomographic image 702 using the MVF consisting of the control point parameters.

Thereafter, the processing of S22 to S24 is performed in the same manner until the image reconstruction condition (image reconstruction interval) is changed.

According to the present embodiment, the virtual tomographic images are assumed at a constant interval between adjacent images, and the provisional control point parameters are sequentially calculated. Therefore, the same result as that obtained in a case where the control point parameters are always calculated at a constant image reconstruction interval without depending on the image reconstruction condition can be obtained, and stable motion estimation can be performed. In addition, even in a case where the image reconstruction interval is large, the motion estimation can be performed in full consideration of the continuity in the body axis direction.

According to the method of the present embodiment, since the PAR image is generated for each virtual tomographic image, the amount of calculation is increased. However, since only the PAR image is generated without performing image reconstruction of the virtual tomographic image itself, it is possible to avoid an excessive calculation load.

In the present embodiment, the method of Embodiment 1, that is, the method of adjusting the weight $\lambda_2$ of the regularization term of the time domain of the motion estimation model (Equation (5)) according to the change in the image reconstruction interval can also be used in combination.

Although the embodiments of the processing of mainly the computing unit 30 (processor) of the X-ray CT apparatus according to the present invention have been described above, the processing described as each embodiment and modification example can be appropriately combined as long as there is no technical contradiction, and such a combination is also included in the present invention. In addition, the present invention is effectively applied to asynchronous imaging, but can be similarly applied to synchronous imaging.

Further, the present invention also includes changing known configurations included in the X-ray CT apparatus and the processor of the above-described embodiments or adding the known configurations, as long as the gist of the present invention is not changed.

Explanation of References

1: X-ray CT apparatus
10: imaging unit
30: computing unit
200: processor
310: image reconstruction unit
311: PAR image generation unit
312: motion correction reconstruction unit
330: motion information acquisition unit
331: motion vector calculation unit
332: motion estimation model adjustment unit

**Claims**

1. An X-ray CT apparatus (1) comprising:

   an imaging unit (10) that has a scanner (100) equipped with an X-ray source (102) and an X-ray detector (103) and rotating around a subject (3) and a moving mechanism (107) moving a position of the scanner in a body axis direction of the subject relative to the subject, and that acquires transmitted X-ray data having different angles with respect to the subject and different positions in the body axis direction; and
   a processor (200) that generates a tomographic image (530) of the subject using the transmitted X-ray data acquired by the imaging unit,
   wherein the processor is configured to generate a pair of partial reconstruction images (510) at positions directly facing each other using the transmitted X-ray data, acquire motion information (520) of the subject during scanning by applying a motion estimation model to the pair of partial reconstruction images, and reconstruct the tomographic image using the motion information and the transmitted X-ray data acquired in an angle range of 180 degrees or more,
   the motion estimation model includes a first regularization term that maintains spatial continuity of an image and a second regularization term that maintains temporal continuity of the image, which are independently adjustable, and
   the processor automatically changes at least one of the motion estimation model or a calculation method of motion estimation using the motion estimation model according to an image reconstruction condition.

2. The X-ray CT apparatus according to claim 1,
   wherein the processor adjusts the motion estimation model according to the image reconstruction condition.

3. The X-ray CT apparatus according to claim 2,
   wherein the processor adjusts at least one of a weight of the first regularization term or a weight of the second regularization term according to the image reconstruction condition.

4. The X-ray CT apparatus according to claim 3,
   wherein the processor adjusts the weight of the first regularization term according to a field of view (FOV) set as the image reconstruction condition.

5. The X-ray CT apparatus according to claim 3 or 4,
   wherein the processor adjusts the weight of the second regularization term according to an image reconstruction interval set as the image reconstruction condition.

6. The X-ray CT apparatus according to claim 3, 4 or 5,
   wherein the processor adjusts a control point of the motion estimation model in addition to adjustment of a weight of a regularization term according to the image reconstruction condition.

7. The X-ray CT apparatus according to any one of claims 2-6,
   wherein the processor adjusts a control point of the motion estimation model according to a field of view (FOV) set as the image reconstruction condition.

8. The X-ray CT apparatus according to claim 7,
   wherein the processor adjusts the number of pixels between adjacent control points of the motion estimation model according to the FOV.

9. The X-ray CT apparatus according to claim 7 or 8,
    wherein the processor adjusts coordinates of the control point such that a position of the control point with respect to an image center in an image space is constant according to the FOV

10. The X-ray CT apparatus according to any preceding claim,
    wherein the processor dynamically adjusts a calculation method according to an image reconstruction interval set as the image reconstruction condition in calculation of a control point parameter using the motion estimation model.

11. The X-ray CT apparatus according to claim 10,
    wherein the processor sets one or more virtual cross sections at a constant interval between a cross section of interest and an adjacent cross section adjacent to the cross section of interest in the body axis direction of the subject, executes the calculation of the control point parameter using the partial reconstruction image in the virtual cross section until a position of the virtual cross section reaches a position of the adjacent cross section, and sets the control point parameter calculated in the virtual cross section in a case where the position of the virtual cross section reaches the position of the adjacent cross section as the control point parameter of the adjacent cross section.

12. A processor (200) for image processing that processes transmitted X-ray data acquired by an X-ray CT apparatus (1), the processor being configured to:

    generate a pair of partial reconstruction images (510) at positions directly facing each other using the transmitted X-ray data;
    acquire motion information (520) of a subject during scanning by applying a motion estimation model including a first regularization term that maintains spatial continuity of an image and a second regularization term that maintains temporal continuity of the image, which are independently adjustable, to the pair of partial reconstruction images; and
    reconstruct a tomographic image (530) using the motion information and the transmitted X-ray data acquired in an angle range of 180 degrees or more.

13. The processor according to claim 12,
    wherein in a case of acquiring the motion information, at least one of the motion estimation model used for acquisition of the motion information or a calculation method using the motion estimation model is dynamically changed according to an image reconstruction condition which is set upon motion correction reconstruction.

# FIG. 1

EP 4 589 538 A1

FIG. 2

EP 4 589 538 A1

# FIG. 3

30

## COMPUTING UNIT

310

### IMAGE RECONSTRUCTION UNIT

PAR IMAGE GENERATION UNIT — 311

MOTION CORRECTION
RECONSTRUCTION UNIT — 312

330

### MOTION INFORMATION
ACQUISITION UNIT

MOTION VECTOR
CALCULATION UNIT — 331

MOTION ESTIMATION MODEL
ADJUSTMENT UNIT — 332

# FIG. 4

```
                    ┌──────────────┐
                    │    Start     │
                    └──────┬───────┘
                           │
    ┌──────────────────────────────────────────┐
    │  START SCANNING/ACQUIRE PROJECTION DATA   │────S1
    └──────────────────────┬───────────────────┘
                           │
    ┌──────────────────────────────────────────┐
    │     SET IMAGE RECONSTRUCTION CONDITION    │────S2
    └──────────────────────┬───────────────────┘
                           │
    ┌──────────────────────────────────────────┐
    │           GENERATE PAR IMAGE             │────S3
    └──────────────────────┬───────────────────┘
                           │
    ┌──────────────────────────────────────────┐
    │  ACQUIRE MOTION INFORMATION USING MOTION  │────S4
    │           ESTIMATION MODEL                │
    └──────────────────────┬───────────────────┘
                           │
    ┌──────────────────────────────────────────┐
    │  IMAGE RECONSTRUCTION PROCESSING USING    │────S5
    │           MOTION INFORMATION              │
    └──────────────────────┬───────────────────┘
                           │
                    ┌──────────────┐
                    │     End      │
                    └──────────────┘
```

FIG. 5

PROJECTION DATA 500

PAIR IMAGE PAIR 510

MOTION INFORMATION (MVF) 520

CORRECTED TOMOGRAPHIC IMAGE 530

# FIG. 6

SCANNING

PROJECTION DATA

PARTIAL IMAGE (PAR IMAGE) PAIR

EP 4 589 538 A1

# FIG. 7

701　702　703

t-1-th　　t-th　　t+1-th

→ SPATIAL ADJACENCY INFORMATION

⤳ TEMPORAL ADJACENCY INFORMATION

● CONTROL POINT

✦ CONTROL POINT OF INTEREST

BODY AXIS DIRECTION

EP 4 589 538 A1

FIG. 8

FIG. 9

# FIG. 10

```
                    ┌──────────┐
                    │  Start   │
                    └──────────┘
                          │
    ┌─────────────────────────────────────────────────┐
    │ AUTOMATICALLY SET CONTROL POINT POSITION         │ ⌇S21
    └─────────────────────────────────────────────────┘
                          │
    ┌───▶─────────────────────────────────────────────┐
    │   │ ACQUIRE PARTIAL TOMOGRAPHIC IMAGE PAIR        │ ⌇S22
    │   └─────────────────────────────────────────────────┘
    │                     │
    │   ┌─────────────────────────────────────────────────┐
    │   │ ACQUIRE MOTION INFORMATION                       │ ⌇S23
    │   └─────────────────────────────────────────────────┘
    │                     │
    │             ╱───────────────────╲        S24
    │   No      ╱   REACH               ╲ ⌇
    └─────────◁ TOMOGRAPHIC IMAGE GENERATION ▷
                ╲       POSITION           ╱
                  ╲───────────────────────╱
                          │  Yes
                    ┌──────────┐
                    │   End    │
                    └──────────┘
```

# FIG. 11

701   7011   7012   702

TOMOGRAPHIC IMAGE (t-th)
VIRTUAL TOMOGRAPHIC IMAGE 1
VIRTUAL TOMOGRAPHIC IMAGE 2
· · ·
TOMOGRAPHIC IMAGE (t+1-th)

IMAGE INTERVAL d OF VIRTUAL TOMOGRAPHIC IMAGE

IMAGE INTERVAL D OF TOMOGRAPHIC IMAGE

BODY AXIS DIRECTION

→ SPATIAL ADJACENCY INFORMATION

⤳ TEMPORAL ADJACENCY INFORMATION

● CONTROL POINT

EP 4 589 538 A1

## EUROPEAN SEARCH REPORT

**Application Number**

EP 25 15 1982

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 565 744 B2 (SAMSUNG ELECTRONICS CO LTD [KR]; KOREA ADVANCED INST SCI & TECH [KR]) 18 February 2020 (2020-02-18) | 1-3,6-13 | INV. G06T11/00 A61B6/00 |
| A | * Col. 12, lines 11-13; Col 4, lines 17-20; Col. 5, lines 46-49; Col. 8, lines 41- 43; Col. 10, lines 54-60, Col. 11, lines 48-62, Col 12, Equation 3, Equation 5 * | 4,5 | G06T7/33 |
| A | ROHKOHL CHRISTOPHER ET AL: "Improving best-phase image quality in cardiac CT by motion correction with MAM optimization", MEDICAL PHYSICS, AIP, MELVILLE, NY, US, vol. 40, no. 3, 1 March 2013 (2013-03-01), pages 31901-31901, XP012171041, ISSN: 0094-2405, DOI: 10.1118/1.4789486 [retrieved on 2013-02-08] * Section II.H.2.b * | 6-9 | |
| A | SCHREIBMANN ET AL: "Image interpolation in 4D CT using a BSpline deformable registration model", INTERNATIONAL JOURNAL OF RADIATION: ONCOLOGY BIOLOGY PHYSICS, PERGAMON PRESS, USA, vol. 64, no. 5, 1 April 2006 (2006-04-01), pages 1537-1550, XP005357588, ISSN: 0360-3016, DOI: 10.1016/J.IJROBP.2005.11.018 * figure 1 * | 10,11 | **TECHNICAL FIELDS SEARCHED (IPC)** G06T A61B |
| A | US 2023/329662 A1 (TETSUMURA YUSUKE [JP] ET AL) 19 October 2023 (2023-10-19) * the whole document * | 1,12 | |

The present search report has been drawn up for all claims

| Place of search | Date of completion of the search | Examiner |
|---|---|---|
| Munich | 11 April 2025 | Tibrewal-Fabricius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 1982

11-04-2025

| Patent document cited in search report | Publication date | Patent family member(s) | Publication date |
|---|---|---|---|
| US 10565744 B2 | 18-02-2020 | NONE | |
| US 2023329662 A1 | 19-10-2023 | CN 116898465 A | 20-10-2023 |
| | | JP 2023156873 A | 25-10-2023 |
| | | US 2023329662 A1 | 19-10-2023 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024006305 A

- US 20180005414 A